(19) Europäisches Patentamt
European Patent Office
Office européen des brevets

(11) Veröffentlichungsnummer: **0 000 511**
**B1**

(12) # EUROPÄISCHE PATENTSCHRIFT

(21) Anmeldenummer: **78100382.7**

(22) Anmeldetag: **12.07.78**

(51) Int. Cl.³: **G 03 C 5/54,** C 09 B 29/08, C 07 D 209/00, C 07 C 107/04

(54) **Fotografisches Farbdiffusionsübertragungsverfahren**

(30) Priorität: **22.07.77 DE 2733112**

(43) Veröffentlichungstag der Anmeldung:
**07.02.79 Patentblatt 79/03**

(45) Bekanntmachung des Hinweises auf die Patenterteilung:
**23.07.80 Patentblatt 80/15**

(84) Benannte Vertragsstaaten:
**BE DE FR GB**

(56) Entgegenhaltungen:
**DE - A - 2 339 713**
**FR - A - 2 254 046**
**FR - A - 2 263 232**
**US - A - 3 357 969**

(73) Patentinhaber: **Agfa-Gevaert Aktiengesellschaft Patentabteilung**
**D - 5090 Leverkusen 1 (DE)**

(72) Erfinder: **Stolzenburg, Rudolf, Chem.-Ing.grad.**
**Bogenstrasse 27**
**D - 4018 Langenfeld (DE)**
**Marx, Paul, Dr.**
**Nauener Strasse 25**
**D - 5090 Leverkusen 1 (DE)**
**Püschel, Walter, Dr.**
**Berta-von-Suttner-Strasse 54**
**D - 5090 Leverkusen 1 (DE)**

EP 0 000 511 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Fotografisches Farbdiffusionsübertragungsverfahren

Die Erfindung betrifft ein Verfahren zur Herstellung farbfotografischer Bilder nach dem Farbstoffdiffusionsübertragungsverfahren sowie ein hierfür geeignetes fotografisches Material, das neue diffusionsfeste, farbgebende Verbindungen enthält, die gelbe diffundierende Azofarbstoffe mit einer Cyanalkylaminogruppe in Freiheit setzen.

Unter den bisher bekannt gewordenen Verfahren zur Herstellung farbiger fotographischer Bilder nach dem Farbdiffusionsübertragungsverfahren, gewinnen in jüngster Zeit zunehmend solche an Bedeutung, die auf der Verwendung diffusionsfest eingelagerter farbgebender Verbindungen beruhen, aus denen bei der Entwicklung diffundierende Farbstoffe oder Farbstoffvorläuferprodukte bildmäßig abgespalten und auf eine Bildempfangsschicht übertragen werden.

Zu den heirfür geeigneten, farbgebenden Verbindungen sind beispielsweise die in der DT—PS 1 095 115 beschriebenen nichtdiffundierenden Farbkuppler zu zählen, die bei der Entwicklung als Folge einer Reaktion mit dem Oxidationsprodukt einer aus einem primären aromatischen Amin bestehenden Farbentwicklerverbindung, einen vorgebildeten oder bei der Farbkupplung erzeugten Farbstoff in diffundierender Form in Freiheit setzen. Die Auswahl der benötigten Entwicklerverbindung beschränkt sich hierbei naturgemäß auf Farbentwickler.

Weiterhin ist hierbei auf die in der DT—OS 1 930 215 beschriebenen nichtdiffundierenden farbgebenden Verbindungen hinzuweisen, die über eine spaltbare Hydrazongruppierung einen vorgebildeten latent diffusionsfähigen Farbstoffrest mit einem diffusionsfest machenden Rest verknüpft enthalten. Diese Verbindungen sind nicht also Farbkuppler zu bezeichnen, und es hat sich auch erwiesen, daß die Auswahl der Entwicklerverbindungen, die zur Freisetzung des diffundierenden Farbstoffrestes erforderlich sind, keineswegs auf die üblichen Farbentwickler beschränkt ist, sondem daß auch Schwarz-Weißentwickler z.B. Brenzkatechine sehr gut brauchbar sind.

In der DT—OS 1 772 929 sind ferner nichtdiffundierende farbige Verbindungen mit einer besonderen Gruppierung beschrieben, die bei der Entwicklung eine oxidative Ringschlußreaktion eingehen und hierbei einen vorgebildeten Farbstoffrest in diffundierender Form in Freiheit setzen. Die dort vorgestellten Verbindungen lassen sich in zwei Gruppen einteilen. Die Verbindungen der einen Gruppe benötigen zur Entwicklung eine übliche Farbentwicklerverbindung, mit deren Oxidationsprodukt sie kuppeln und in einer nachfolgenden Ringschlußreaktion den vorgebildeten Farbstoffrest in diffundierender Form in Freiheit setzen. Die Verbindungen der anderen Gruppe stellen selbst Silberhalogenidentwicklungsmittel dar und vermögen daher auch in Abwesenheit weiterer Entwicklerverbindungen in der oxidierten Form die vorerwähnte Ringschlußreaktion unter Freisetzung der diffundierenden Farbstoffe einzugehen.

Schließlich seien an dieser Stelle noch erwähnt die nichtdiffundierenden farbgebenden Verbindungen der DT—OS 2 242 762. Hierbei handelt es sich um Sulfonamidophenole und Sulfonamidoaniline, die nach der bei der Entwicklung erfolgten Oxidation unter dem Einfluß des Entwickleralkalis unter Freisetzung diffundierender Farbstoffe mit einer freien Sulfamoylgruppe gespalten werden.

Die obenerwähnten farbgebenden Verbindungen arbeiten ausnahmslos negativ, d.h. die bildmäßige Verteilung des freigesetzten diffundierenden Farbstoffes entsteht bei Verwendung üblicher (negativ arbeitender) Silberhalogenidemulsionen in Übereinstimmung mit dem bei der Entwicklung erzeugten negativen Silberbild. Zur Erzeugung positiver Farbstoffbilder bedarf es daher der Verwendung von direktpositiven Silberhalogenidemulsionen oder andernfalls der Anwendung eines geeigneten Umkehrverfahrens.

Aus den DT—OSen 2 402 900 und 2 543 902 sind weiterhin nichtdiffundierende farbgebende Verbindungen bekannt, die in nicht oxidierter Form zu einer Spaltungsreaktion unter alkalischen Entwicklungsbedingungen befähigt sind, wobei ein diffundierender Farbstoff in Freiheit gesetzt wird, und bei denen andererseits in oxidierter Form die oben erwähnte Spaltungsreaktion erschwert oder verhindert wird. Derartige Verbindungen sind in Kombination mit herkömmlichen Negativemulsionen zur Herstellung positiver Übertragsfarbbilder geeignet.

Es ist schwierig unter den bisher bekannten farbgebenden Verbindungen geeignete auszusuchen, die in jeder Hinsicht zufriedenstellend sind, und zwar sowohl einerseits hinsichtlich ihrer ausreichenden Reaktivitat und andererseits hinsichtlich ihrer ausreichenden Stabilität. Sie sollen die diffundierenden Farbstoffe nicht bereits bei der alkalischen Entwicklung in Freiheit setzen, sondern erst, nachdem eine bildmäßige Oxidation durch das bildmäßig entwickelte Silberhalogenid stattgefunden hat. Andererseits muß die Freisetzung der diffundierenden Farbstoffe entweder aus der oxidierten oder aus der nicht oxidierten Form der farbgebundenen Verbindungen rasch genug erfolgen und ebenso ist ein schneller Übertrag der diffundierenden Farbstoffe erforderlich.

Sehr wichtig ist, daß die Farbstoffe in der Bildempfangsschicht in ausreichendem Maße festgelegt werden können und daß sie außerdem ausgezeichnete spektrale Eigenschaften und hervorragende Stabilität gegen Licht und Wärme aufweisen.

In der DT—OS 2 626 821 sind gelbe Farbstoffe liefernde farbgebende Verbindungen für das Farbdiffusionsübertragungsverfahren beschrieben, die sich durch besonders vorteilhafte Absorptionsspektren auszeichnen sollen sowie eine ausgezeichnete Farbstabilität aufweisen sollen. Es wurde jedoch festgestellt, daß die aus diesen farbgebenden Verbindungen freigesetzten gelben Farbstoffe hinsichtlich ihrer Stabilität gegenüber Licht sehr zu wünschen übrig lassen.

Der Erfindung liegt daher die Aufgabe zugrunde neue farbgebende Verbindungen für das Farbdiffusionsübertragsverfahren anzugeben, aus denen bei der fotografischen Entwicklung diffundierende gelbe Farbstoffe in Freiheit gesetzt werden, die erwünschte spektrale Eigenschaften sowie eine gute Beizenfestigkeit aufweisen, und die sich ferner durch eine verbesserte Stabilität gegenüber Licht auszeichnen.

Gegenstand der vorliegenden Erfindung ist ein fotografisches Farbdiffusionsübertragungsverfahren zur Herstellung farbiger Bilder, bei dem ein fotografisches Material mit mindestens einer lichtempfindlichen Silberhalogenidemulsionsschicht und einer dieser zugeordneten nichtdiffundierenden farbgebenden Verbindung bildmäßig belichtet und mit einem Silberhalogenidentwicklungsmittel entwickelt wird, wobei durch das Entwickleralkali bildmäßig ein diffundierender Farbstoff aus der nichtdiffundierenden farbgebenden Verbindung abgespalten und auf eine Bildempfangsschicht übertragen wird. Das Verfahren ist dadurch gekennzeichnet, daß als nichtdiffundierende farbgebende Verbindung eine Verbindung der folgenden Formel I verwendet wird:

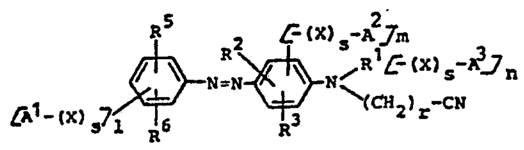

(I)

worin bedeuten

$A^1$ einen gegebenenfalls über ein Bindeglied X angeknüpften einen diffusionsfestmachenden Rest enthaltenden oxidierbaren organischen Trägerrest in o-, m- oder p-Stellung zur Azogruppe, aus dem unter den Bedingungen der fotografischen Entwicklung entweder in oxidierter Form oder in nicht oxidierter Form durch Entwickleralkali mindestens ein Teil zusammen mit dem diffusionsfestmachenden Rest abgespalten wird unter gleichzeitiger bildmäßiger Freisetzung eines diffundierenden Azofarbstoffes;

$A^2$ einen gegebenenfalls über ein Bindeglied X angeknüpften einen diffusionsfestmachenden Rest enthaltenden oxidierbaren organischen Trägerrest, der entweder direkt oder über einen der Substituenten $R^2$ und $R^3$ in o- oder m-Stellung an den die Cyanalkylaminogruppe tragenden Phenylring angeknüpft ist und aus dem unter den Bedingungen der fotografischen Entwicklung entweder in oxidierter Form oder nichtoxidierter Form durch Entwickleralkali mindenstens ein Teil zusammen mit dem diffusionsfest machenden Rest abgespalten wird unter gleichzeitiger bildmäßiger Freisetzung eines diffundierenden Azofarbstoffes;

$A^3$ einen gegebenenfalls über ein Bindeglied X angeknüpften, einen diffusionsfest machenden Rest enthaltenden oxidierbaren organischen Trägerrest, der mit dem Substituenten $R^1$ verknüpft ist und aus dem unter den Bedingungen der fotografischen Entwicklung entweder in oxidierter Form oder in nichtoxidierter Form durch Entwickleralkali mindestens ein Teil zusammen mit dem diffusionsfestmachenden Rest abgespalten wird, unter gleichzeitiger bildmäßiger Freisetzung eines diffundierenden Azofarbstoffes;

$R^1$ Wasserstoff, Alkyl mit 1 bis 5 C-Atomen, Aralkyl oder Aryl;

$R^2$ und $R^3$ (gleich oder verschieden), je einen der unter $R^1$ genannten Reste, Sulfo, $-COOR^4$, Halogen, Trihalogenmethyl, Acylamino, Acyloxy (wobei die genannten Acylreste sich ableiten von aliphatischen oder aromatischen Carbon- oder Sulfonsäuren, einschließlich aliphatischer und aromatischer Carbaminsäuren und Kohlensäuremonoester), ferner Carbamoyl, Sulfamoyl, $-OR^4$, $-SR^4$, $-SOR^4$, $-SO_2-R^4$, (wobei $R^4$ eine der für $R^1$ angegebenen Bedeutungen hat), $-NO_2$ oder $-CN$;

$R^5$ und $R^6$ (gleich oder verschieden) je einen Substituenten der für $R^2$ und $R^3$ angegebenen Art, vorzugsweise Wasserstoff, Halogen wie Chlor, Alkyl wie Methyl, Alkoxy wie Methoxy, Acylamino wie Acetamino oder Nitro;

X ein bivalentes Bindeglied der Formel

$$-R-(L)_p-(R)_q-,$$

worin R einen Alkylenrest mit 1 bis 6 C-Atomen oder einen gegebenenfalls substituierten Phenylrest bedeutet und wobei die beiden Reste R gleiche oder verschiedene Bedeutungen haben können;

L $-O-$, $-CO-$, $-CONR^7$, $-SO_2NR^7$, $-O-CO-NR^7$, $-S-$, $-SO_2-$, $-SO-$ bedeutet ($R^7$ = Wasserstoff oder Alkyl);

p 0 oder 1

q 0 oder 1, wobei q = 1, falls p = 1

s 0 oder 1

l, m, n jeweils 0 oder 1 mit der Maßgabe, daß l + m + n = 1

r eine ganze Zahl von 1 bis 4, vorzugsweise 2 oder 3.

Die erfindungsgemäßen farbgebenden Verbindungen enthalten somit einen Azofarbstoffrest der Formel

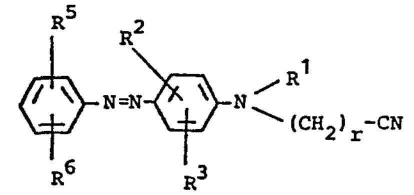

der entweder über den Rest $R^1$ oder über einen der beiden dargestellten Benzolringe mit einem diffusionsfestmachenden oxidierbaren organischen Trägerrest verbunden ist. Dieser Trägerrest ist dabei von solcher Art, daß er entweder in oxidierter Form oder in nichtoxidierter Form unter den Bedingungen der fotografischen Entwicklung durch Entwickleralkali aus den farbgebenden Verbindungen abgespalten wird, so daß diffundierende Azofarbstoffe in Freiheit gesetzt werden. Trägerreste $A^1$, $A^2$, $A^3$ mit derartigen Funktionen sind bekannt. Hier sei beispielsweise auf die in der eingangs erwähnten DT—OS 2 242 762 beschriebenen Sulfonamidophenole und Sulfonamidoaniline hingewiesen, die nach der bei der Entwicklung erfolgten Oxidation unter Einfluß des Entwickleralkalis unter Freisetzung diffundierender Farbstoffe mit einer freien Sulfonamidgruppe gespalten werden. Weiterhin sei beispielsweise verwiesen auf die in DT—OS 2 505 248 und in der deutschen Patentanmeldung P 2 645 656.4 beschriebenen Verbindungen, z.B. die 3-Sulfonamidoindolverbindungen, die in ähnlicher Weise, wenn sie oxidiert sind, einer Spaltung durch das Entwickleralkali unterliegen und hierbei diffundierende Farbstoffe in Freiheit setzen. Auch diese Verbindungen ermöglichen somit einen Farbübertrag an Stellen, in denen eine Entwicklung stattfindet. Weiterhin müssen an dieser Stelle die DT—OSen 2 402 900 und 2 543 902 erwähnt werden. In diesen beiden Offenlegungsschriften sind farbgebende Verbindungen beschrieben, die in einer Spaltungsreaktion unter alkalischen Entwicklungsbedingungen aus der nichtoxidierten Form diffundierende Farbstoffe in Freiheit setzen und bei denen andererseits in oxidierter Form die oben erwähnte Spaltungsreaktion erschwert oder verhindert wird. Derartige Verbindungen ermöglichen somit einen Farbübertrag nur an solchen Stellen, in denen nicht eine durch die Entwicklung bedingte Oxidation stattgefunden hat. Sie sind somit zur Herstellung positiver Übertragsbilder geeignet.

Besonders bevorzugte Verbindungen der vorliegenden Erfindung entsprechen der folgenden Formel II:

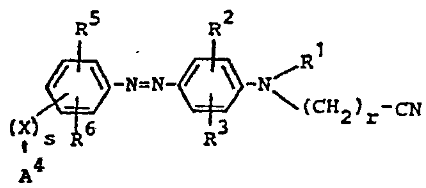

(II)

worin die Symbole $R^1$, $R^2$, $R^3$, $R^5$, $R^6$, X, r und s die bereits angegebene Bedeutung haben und $A^4$ den mit mindestens einem diffusionsfestmachenden Rest substituierten Rest einer der folgenden Formeln bedeutet:

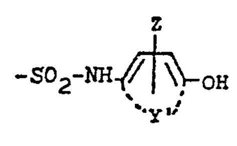

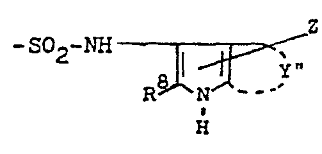

worin

Z für einen diffusionsfestmachenden Rest steht

Y' für einen zur Vervollständigung eines Benzol- oder Naphthalinringes erforderlichen Rest steht

Y" für einen zur Vervollständigung eines ankondensierten, gegebenenfalls substituierten carbocyclischen oder heterocyclischen Ringes steht, und

$R^8$ Wasserstoff, Alkyl, Alkoxy, Aryl, eine heterocyclische Gruppe Carboxyl, Carbamoyl oder Alkoxycarbonyl bedeutet.

$A^4$ stellt somit in der bevorzugten Ausführungsform der vorliegenden Erfindung zusammen mit der verknüpfenden $—NH—SO_2—$ Gruppierung einen nicht diffundierenden oxidierbaren organischen Trägerrest dar, und zwar einen solchen Trägerrest, der nur in oxidierter Form einer Spaltung durch das Entwickleralkali unterliegt. Eine Farbstoffdiffusion findet daher nur an den Stellen im fotografischen Material statt, in denen eine Silberhalogenidentwicklung erfolgt. Farbgebende Verbindungen mit Trägerresten der durch $A^4$ dargestellten Art sind beschrieben in DT—OS 2 242 762, in DT—OS 2 505 248 und der deutschen Patentanmeldung P 2 645 656.4.

Das in den allgemeinen Formeln I und II dargestellte bivalente Bindeglied X kann beispielsweise ein Rest einer der folgenden Formeln sein:

$-CH_2-CH_2-O-CH_2-CH_2-$, 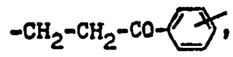 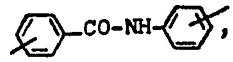 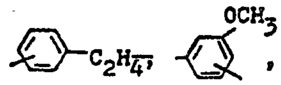

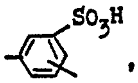 $-CH_2-CH_2-SO_2-C_6H_{12}-$ und $-C_3H_6-NH-CO-C_2H_4-$.

Est ist darauf hinzuweisen, daß die erfindungsgemäßen farbgebenden Verbindungen als intakte Moleküle in den Schichten des fotografischen Materials nicht diffundieren sollen. Zu diesem Zweck enthalten sie einen diffusionsfest machenden Rest, z.B. den Rest Z.

Ausreichende Diffusionsfestigkeit der farbgebenden Verbindungen kann bereits selbst dann gegeben sein, wenn die farbgebenden Verbindungen keine längeren Alkylreste enthalten, da auch dann das Molekül je nach Farbstoffrest eine hinreichende Größe haben kann. Andernfalls besteht die Möglichkeit, die farbgebenden Verbindungen durch Auswahl von Resten geeigneter Größe genügend diffusionsfest zu machen.

Als diffusionsfestmachende Reste sind solche Reste anzusehen, die es ermöglichen, die erfindungsgemäßen Verbindungen in den üblicherweise bei fotografischen Materialien verwendeten hydrophilen Kolloiden diffusionsfest einzulagern. Hierzu sind vorzugsweise organisch Reste geeignet, die im allgemeinen geradkettige oder verzweigte aliphatische Gruppen und gegebenenfalls auch isocyclische oder heterocyclische oder aromatische Gruppen mit im allgemeinen 8 bis 20 C-Atomen enthalten. Mit dem übrigen Molekülteil sind diese Reste entweder direkt oder indirekt, z.B. über eine der folgenden Gruppen verbunden: —NHCO—, —$NHSO_2$—, —NR—, wobei R Wasserstoff oder Alkyl bedeutet, —O— oder —S—. Zusätzlich kann der diffusionsfestmachende Rest auch wasserlöslichmachende Gruppen enthalten, wie z.B. Sulfogruppen oder Carboxylgruppen, die auch in anionischer Form vorliegen können. Da die Diffusionseigenschaften von der Molekülgröße der verwendeten Gesamtverbindung abhängen, genügt es in bestimmten Fällen, z.B. wenn das verwendete Gesamtmolekül groß genug ist, als "diffusionsfestmachende Reste" auch kürzerkettige Reste zu verwenden.

Bei der Entwicklung wird aus den erfindungsgemäßen nichtdiffundierenden farbgebenden Verbindungen mindestens ein Teil des Trägerrestes zusammen mit dem diffusionsfestmachenden Rest abgespalten unter Freisetzung eines diffundierenden Azofarbstoffes. Gleichzeitig kann sich in dem abgespaltenen Trägerrest eine chemische Umwandlung vollziehen, z.B. eine intramolekulare Umlagerung etwa in Form einer Ringschlußreaktion.

Die freigesetzten diffundierenden Azofarbstoffe weisen beispielsweise im Fall der nicht diffundierenden farbgebenden Verbindungen der Formel II eine freie Sulfamoylgruppe auf und entsprechen der folgenden Formel III

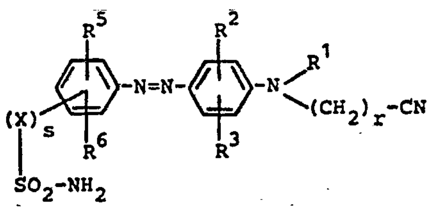

(III)

wobei $R^1$, $R^2$, $R^3$, $R^5$, $R^6$, r und s die bereits angegebene Bedeutung haben.

Beispiele für geeignete farbgebende Verbindungen gemäß der vorliegenden Erfindung sind die folgenden:

$A^5$ =

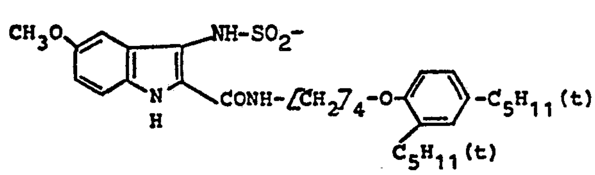

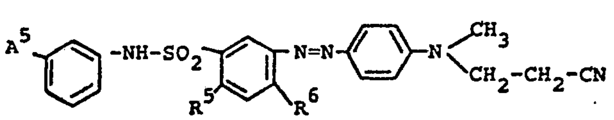

| Verbindung Nr. | $R^5$ | $R^6$ | λ max [nm] |
|---|---|---|---|
| 1 | H | H | 424 |
| 2 | H | Cl | 436 |
| 3 | H | $CH_3$ | 423 |
| 4 | H | $OCH_3$ | 426 |
| 5 | Cl | H | 430 |
| 6 | $CH_3$ | H | 417 |

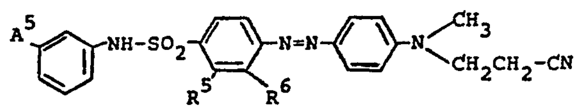

| Verbindung Nr. | $R^5$ | $R^6$ | λ max [nm] |
|---|---|---|---|
| 7 | H | H | 431 |
| 8 | Cl | H | 447 |
| 9 | $CH_3$ | H | 433 |

Verbindung Nr. 10

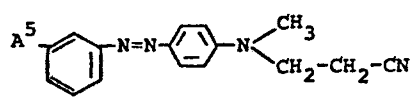

$\lambda$max: 420 nm

Herstellung von Verbindung Nr. 2

a) 3-(4-N-Methyl-N-cyanäthyl-aminobenzol-azo)-4-chlorbenzolsulfonsäure

Zu einer Suspension von 69,2 g 3-Amino-4-chlorbenzolsulfonsäure (90%ig) in 500 ml $H_2O$ und 90 ml konzentrierter Salzsäure wurde eine Lösung von 20,7 g Natriumnitrit in 60 ml $H_2O$ bei 0—5°C zugetropft. Die Suspension wurde 30 Min. nachgerührt. Überschüssiges Nitrit wurde mit Amidosulfonsäure zerstört. Die Suspension wurde bei 5—10°C eingetragen in eine Lösung von 48 g 3-(N-Methylanilino)-propionsäurenitril in 240 ML Aceton. Anschließend wurde der pH-Wert mit 20%iger Natriumacetatlösung auf 3,5 eingestellt. Es wurde 3 Stunden bei 5—10°C und anschließend ohne Kühlung über Nacht nachgerührt. Der Farbstoff wurde abgesaugt, mit 10%iger Kochsalzlösung gewaschen und getrocknet. Ausbeute 93 g; Fp.: >225° (Z).

b) 3-(4-N-Methyl-N-cyanäthyl-aminobenzol-azo)-4-chlorbenzolsulfonsäurechlorid

In eine Vorlage von 140 ml Dimethylformamid wurden 22,8 ml $POCl_3$ eingetragen. Die Lösung wurde auf 25°C gekühlt und 19 g 3-(4-N-Methyl-N-cyanäthyl-aminobenzol-azo)-4-chlorbenzolsulfonsäure wurden bei 25°C eingetragen. Es wurde 1 Stunde bei 25°C nachgerührt. Anschließend wurde der Ansatz auf Eis ausgetragen und der ausfallende Neiderschlag abgesaugt, mit Wasser gewaschen und getrocknet. Ausbeute 18 g; Fp.: >155° (Z).

c) Verbindung Nr. 2.

6,48 g der Verbindung der folgenden Formel

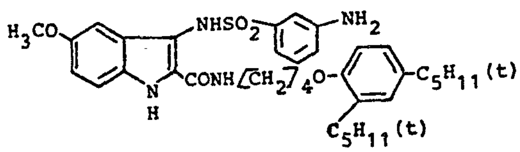

die durch Unsetzung von 2-(2,4-Ditert.-pentylphenoxybutylamino-carbonyl)-3-amino-5-methoxy-indol (dessen Darstellung nach einer in der deutschen Patentanmeldung P 2 645 656.4 beschriebenen Methode erfolgt) mit 3-Nitrobenzolsulfonsäurechlorid und anschließend katalytischer Hydrierung hergestellt wurde, wurde mit 100 ml Chloroform und 3,2 ml Pyridin angeschlämmt und mit 4,4 g 3-(N-Methyl-N-cyanäthyl-aminobenzol-azo)-4-chlorbenzolsulfonsäurechlorid versetzt. Der Ansatz wurde 10 Stunden bei 40°C gerührt. Anschließend wurde der Ansatz gekühlt und unter Zugabe von 350 ml Methanol 30 Minuten gerührt. Der ausfallende Niederschlag wurde abgesaugt, mit Methanol gewaschen und getrocknet.

Ausbeute 6,1 g; Fp.: 244—246° (Z).

In analoger Weise können auch die übrigen farbgebenden Verbindungen der vorliegenden Erfindung hergestellt werden.

Für die Absorptionsmessung und Lichtechtheitsprüfung (siehe Beispiel 2) werden die aus den erfindungsgemäßen farbgebenden Verbindungen abgespaltenen diffundierenden Farbstoffe verwendet. Beispielhaft sei an dieser Stelle die Herstellung des aus Verbindung 2 freigesetzten farbstoffes beschrieben.

Farbstoff aus Verbindung 2:

1,73 g 3-Aminobenzolsulfonsäureamid wurden in 40 ml Chloroform und 3,2 ml Pyridin angeschlämmt und mit 4,4 g 3-(4-N-Methyl-N-cyanäthyl-aminobenzol-azo)-4-chlorbenzolsulfonsäurechlorid (90%ig) versetzt. Es wurde 4 h bei 40°C nachgerührt. Es wurden weitere 0,4 g Sulfochlorid zugesetzt und erneut 3 Stunden bei 40°C gerührt. Der ausfallende Neiderschlag wurde abgesaugt und mit Chloroform gewaschen und getrocknet. Das Nutschgut wurde mit 2 n Salzsäure verrührt, abgesaugt, mit 2 n Salzsäure und $H_2O$ gewaschen. Anschließend wurde die Substanz durch mehrfaches Extrahieren mit Essigester und Einengen der Mutterlauge gereinigt. Ausbeute 3,5; Fp.: 120—122° (Z).

Die erfindungsgemäßen Verbindungen sind gelb gefärbt. Sie werden den Gießlösungen für die Schichten des fotografischen Materials nach einer der üblichen Methoden einverleibt. Die pro Liter Gießlösung verwendete Menge an farbgebender Verbindung variiert in relativ weiten Grenzen, wobei die günstigste Konzentration anhand einfacher Versuche festgestellt wird. Beispielsweise werden pro Liter Gießlösung 5 bis 80 g, vorzugsweise 20 bis 40 g, farbgebende Verbindung verwendet.

Die zur Erzielung des gewünschten Effektes erforderliche Zuordnung zwischen diffusionsfester farbgebender Verbindung und Silberhalogenid kann beispielsweise dadurch hergestellt werden, daß die diffusionsfesten Verbindungen unter Ausnutzung vorhandener wasserlöslichmachender Gruppen aus wäßrig-alkalischen Losungen in die Gießlösungen eingebracht werden. Die nicht-diffundierenden farbgebenden Verbindungen können aber auch in die Schichten nach einem der bekannten Emulgierverfahren eingearbeit werden. Solche Verfahren sind beispielsweise in den englischen Patentschriften 791 219 und 1 099 414 bis 1 099 417 beschrieben. Weiterhin ist es möglich, wäßrige Dispersionen der farbgebenden Verbindungen herzustellen und den jeweiligen Gießlösungen zuzusetzen. Hier zu werden wäßrige Aufschlämmungen der farbgebenden Verbindung beispielsweise durch intensives Rühren unter Zusatz von scharfkantigem Sand oder durch Anwendung von Ultraschall fein vermahlen. In einer weiteren Ausführungsform kann es beispielsweise erwünscht sein, die farbgebenden Verbindungen gemeinsam mit Silberhalogenid und gegebenenfalls Entwicklersubstanzen in Form sogenannter Mikrokapseln in die Schicht einzulagern, wobei auch zwei oder mehr verschieden sensibilisierte lichtempfindliche Silberhalogenid-Emulsionen und die entsprechenden diffusionsfesten Verbindungen in einer einzigen Schicht nach der Art der sogenannten Mischkorn-Emulsionen vereinigt werden, wie dies beispielsweise in der amerikanischen Patentschrift 2 698 794 beschrieben ist. Die nicht diffundierenden farbgebenden Verbindungen können in einer lichtempfindlichen Schicht selbst oder in einer Nachbarschicht untergebracht sein. Beispielsweise ist der rotempfindlichen Schicht eine einen blaugrünen Farbstoff abspaltende Verbindung, der grünempfindlichen Schicht eine einen purpurnen Farbstoff abspaltende Verbindung, und der blauempfindlichen Schicht eine der erfindungsgemäßen einen gelben Farbstoff abspaltende farbgebende Verbindung zugeordnet.

Unter "Zuordnung" und "zugeordnet" wird verstanden, daß die gegenseitige Anordnung von Silberhalogenidemulsion und farbgebenden Verbindung von solcher Art ist, daß ein Wechselwirkung zwischen ihnen möglich ist, die eine bildmäßige Übereinstommung zwischen gebildetem Silberbild (bei negativ arbeitenden farbgebenden Verbindungen) bzw. nicht entwickeltem Silberhalogenid (bei positiv arbeitenden farbgebenden Verbindungen) einerseits und bildmäßiger Verteilung des freisetzenden, diffundierenden Farbstoffes andererseits zuläßt. Zweckmäßigerweise wird hierbei die zugeordnete, farbgebende Verbindung in die Silberhalogenidemulsion selbst oder in eine zu der Silberhalogenidemulsionsschicht benachbarte Schicht eingelagert, wobei diese benachbarte Schicht vorzugsweise (gesehen in Richtung des bei der Belichtung einfallenden Lichtes) hinter der Silberhalogenidemulsionsschicht liegt. Die erfindungsgemäßen farbgebenden Verbindungen werden bei der Entwicklung des Silberbildes von Entwickleroxidationsprodukten bildmäßig oxidiert und unterliegen dann unter dem Einfluß des Entwickler- bzw. Aktivatoralkalis einer Spaltungsreaktion, bei der die Farbstoffreste in diffundierender Form in der Regel also Farbstoffsulfonamide in Freiheit gesetzt werden. Zur Entwicklung sind die üblichen fotografischen Entwicklerverbindungen geeignet, soweit sie in der Lage sind in oxidierter Form die erfindungsgemäßen farbgebenden Verbindungen zu oxidieren. Beispiele für geeignete Entwickler sind folgende:

Hydrochinon
N-Methylaminophenol
1-Phenyl-3-pyrazolidon
1-Phenyl-4,4-dimethyl-3-pyrazolidon
1-Phenyl-4-methyl-4-hydroxymethyl-3-pyrazolidon
Aminophenole
N,N-Diäthyl-p-phenylendiamin
N-Äthyl-N-hydroxyäthyl-p-phenylendiamin
N-Äthyl-N-$\omega$-sulfobutyl-p-phenylendiamin
3-Methyl-N,N-diäthyl-p-phenylendiamin
N,N,N',N'-Tetraalkyl-p-phenylendiamine wie Tetramethyl-p-phenylendiamin, 1,4-Bis-pyrrolidinobenzol, Reduktone.

Es ist besonders darauf hinzuweisen, daß die Auswahl an Entwicklersubstanzen bei dem erfindungsgemäßen Verfahren nicht auf Farbentwickler beschränkt ist, sondern daß auch übliche Schwarsweißentwickler zur Anwendung gelangen können, was wegen der geringeren Verfärbungsneigung der letzteren als Vorteil anzusehen ist. Die Entwickler können bereits in den Schichten des farbfotografischen Materials enthalten sein, wo sie durch die alkalische Aktivatorflüssigkeit aktiviert werden, oder in der alkalischen Verarbeitungsflüssigkeit oder -paste. Da die erfindungsgemäßen farbgebenden Verbindungen selbst Entwicklereigenschaften haben, kann in einzelnen Fällen auf die Verwendung von Hilfsentwicklerverbindungen verzichtet werden. In diesem Fall wird die farbgebende Verbindung unmittelbar durch entwickelbares Silberhalogenid oxidiert.

Wenn die bildmäßige Verteilung des bei der Entwicklung freigesetzten diffundierenden Farbstoffes mit dem entwickelten Silberbild übereinstimmt, wie dies der Fall ist bei den farbgebenden Verbindungen des Typs wie er in den DT—OSen 2 242 762, 2, 505 248 und auch der DT—OS 1 772 929 beschrieben ist, bedarf es zur Erzeugung positiver farbiger Übertragungsbilder der Verwendung von direktpositiven Silberhalogenidemulsionen oder, falls übliche Negativemulsionen verwendet werden, der Anwendung eines geeigneten Umkehrverfahrens. Im Falle der farbgebenden Verbindungen der zuletzt genannten DT—OS werden die diffundierenden Farbstoffe zwar nicht unmittelbar als Folge einer Spaltung durch Alkali, sondern eher also Folge einer intramolekularen Verdrängsgungsreaktion unter Ringschluß in Freiheit gesetzt. Auch weisen die freigesetzten Farbstoffe nicht eine freie Sulfamoylgruppe auf wie die aus den erfindungsgemäß bevorzugt verwendeten farbgebenden Verbindungen abgespaltenen Farbstoffe, sondern eine Sulfinsäuregruppe. Jedoch ist die Erfindung keineswegs beschränkt auf solche farbgebende Verbindungen, bei denen die Spaltung durch Alkali erfolgt.

Ein solches Umkehrverfahren steht in dem Silbersalzdiffusionsverfahren zur Verfügung. Die fotografische Umkehrung mit Hilfe des Silbersalzdiffusionsverfahrens zur Erzeugung von positiven farbigen Bildern unter Verwendung konventioneller Farbkuppler ist beispielsweise in der US—PS 2 763 800 beschrieben. Durch Austausch der Farbkuppler gegen die erwähnten farbgebenden Verbindungen, kommt man zu einem lichtempfindlichen Element, das für das Farbstoffdiffusionsübertragungsverfahren geeignet ist. Ein solches lichtempfindliches Element weist beispielsweise mindestens eine Kombination auf aus einer lichtempfindlichen Silberhalogenidemulsionsschicht und einer dieser zugeordneten Bindemittelschicht, die Entwicklungskeime für die physikalische Entwicklung und eine farbgebende Verbindung enthält.

Bei der Entwicklung wird in der lichtempfindlichen Silberhalogenidemulsionsschicht der belichtete Anteil des Silberhalogenids chemisch entwickelt; der nicht belichtete Anteil wird vermittels eines Silberhalogenidlösungsmittels in die zugeordnete Entwicklungskeime enthaltende Bindemittelschicht übertragen und dort physikalisch entwickelt. Wenn für die physikalische Entwicklung ein Entwickler verwendet wird, der in oxidierter Form als Folge einer Reaktion mit der in dieser Schicht vorhandenen farbgebenden Verbindung einen diffundierenden Farbstoff in Freiheit zu setzen vermag, dann kommt es zur Bildung einer bildmäßigen Verteilung diffundierender Farbstoffe, die auf eine Bildempfangsschicht übertragen werden können und dort ein positives farbiges Bild bilden.

Bei der Umkehrung unter Verwendung von bildmäßig Entwicklungsinhibitoren abspaltenden Verbindungen besteht das lichtempfindliche Element aus mindestens einer Schichtkombination einer lichtempfindlichen Silberhalogenidemulsionsschicht und einer zweiten unbelichtet entwickelbaren Emulsionsschicht, die die farbgebende Verbindung enthält. Die lichtempfindliche Silberhalogenidemulsionsschicht wird mit beispielsweise Farbentwicklern in Gegenwart von bestimmten Verbindungen entwickelt, die bei der Reaktion mit oxidiertem Farbentwickler entwicklungshemmende Substanzen abspalten. Die in der lichtempfindlichen Schicht bildmäßig in Freiheit gesetzten entwicklungshemmenden Substanzen diffundieren in die benachbarte unbelichtet entwickelbare Emulsionsschicht

und hemmen dort bildmäßig die Entwicklung. Dabei werden die ungehemmten (positiven) Anteile der unbelichtet entwickelbaren Emulsionsschicht durch den restlichen Entwickler entwickelt, dessen Oxidationsprodukte dann mit den nichtdiffundierenden farbgebenden Verbindungen gemäß der Erfindung unter Freisetzung diffundierender Farbstoffe reagieren, die bildmäßig auf das Bildempfangselement übertragen werden. Geeignete Verbindungen, die bei Reaktion mit Farbentwickleroxidationsprodukten entwicklungshemmende Substanzen abspalten, sind beispielsweise die bekannten DIR-Kuppler (DIR = development inhibitor releasing), bei denen es sich um Farbkuppler handelt, die in der Kupplungsstelle einen abspaltbaren Inhibitorrest enthalten. Derartige DIR-Kuppler sind beispielsweise in der US—PS 3 227 554 beschrieben.

Eine weitere Gruppe von Verbindungen, die bei Reaktion mit Farbentwickleroxidationsprodukten entwicklungshemmende Substanzen abspalten, ist in der US—PS 3 632 345 beschreiben. Hierbei handelt es sich nicht um Farbkuppler. Entsprechend entstehen bei der Freisetzung der entwicklungshemmenden Substanzen auch keine Farbstoffe. Gemäß der DT—PS 1 229 389 können bei einem solchen Verfahren schließlich auch geeignete substituierte, nichtdiffundierende Hydrochinon-Verbindungen verwendet werden, die bei der Reaktion mit Entwickleroxydationsprodukten zu den entsprechenden Chinonen oxidiert werden und dabei entwicklungshemmende Mercaptane aspalten.

Als direkt positive Silberhalogenidemulsionen sind prinzipiell alle direkte positiven Silberhalogenidemulsionen geeignet, die bei einer einfachen Entwicklung ein positives Silberbild und eine diesem entsprechende bildmäßige Verteilung von Entwickleroxydationsprodukten erzeugen. In Frage kommen beispielsweise solche Silberhalogenidemulsionen, in denen durch Belichten oder durch chemische Behandlung ein entwickelbarer Schleier erzeugt worden ist, der unter Einhaltung bestimmter bedingungen bei der bildmäßigen Belichtung bildmäßig zerstört wird. An den unbelichteten Stellen bleibt der Schleier erhalten, so daß bei der anschließenden Entwicklung ein direkt positives Silberbild erhalten wird, und in Übereinstimmung damit eine bildmäßige Verteilung an diffundierendem Farbstoff, wenn der direkt positiven Silberhalogenidemulsion eine erfindungsgemäße farbgebende Verbindung zugeordnet ist.

Eine weitere Gruppe von direkt positiven Silberhalogenidemulsionen, die gemäß der vorliegenden Erfindung bevorzugt verwendet werden, umfaßt die sogenannte unverschleierten direkt positiven Silberhalogenidemulsionen, die eine Lichtempfindlichkeit überwiegend im Innern der Silberhalogenidkörner aufweisen. Bei der bildmäßigen Belichtung dieser Emulsionen bildet sich ein latentes Bild überwiegend im Innern der Silberhalogenidkörner. Die Entwicklung derartiger unverschleierter direkt positiver Silberhalogenidemulsionen wird allerdings unter schleiernden Bedingungen vorgenommen, wobei vorwiegend an den unbelichteten Stellen ein Schleier erzeugt und bei der Entwicklung ein positives Silberbild entwickelt wird. Die unverschleierten direkt positiven Silberhalogenidemulsionen sind dadurch charakterisiert, daß belichtete Proben bei der Entwicklung unter Verwendung eines typischen Oberflächenentwicklers der folgenden Zusammensetzung:

| | |
|---|---|
| p-Hydroxyphenylglycin | 10 g |
| Natriumcarbonat (kristallisiert) | 100 g |
| auffüllen mit Wasser auf | 1000 ml |

vorzugsweise kein Silberbild, oder nur eines mit sehr geringer Dichte ergeben, während bei Verwendung eines Innenkeimentwicklers der folgenden Zusammensetzung:

| | |
|---|---|
| Hydrochinon | 15 g |
| Monomethyl-p-aminophenolsulfat | 15 g |
| Natriumsulfit (wasserfrei) | 50 g |
| Kaliumbromid | 10 g |
| Natriumhydroxid | 25 g |
| Natriumthiosulfat (kristallisiert) | 20 g |
| auffüllen mit Wasser auf | 1000 ml |

ein Silberbild mit ausreichender Dichte entsteht.

Die selektive Verschleierung der bildmäßig belichteten unverschleierten Direktpositiv-Emulsionen kann vor oder während der Entwicklung durch Behandlung mit einem Verschleierungsmittel vorgenommen werden. Geeignete Verschleierungsmittel sind reduktionsmittel wie Hydrazin oder substituierte Hydrazine. Verwiesen sei beispielsweise auf US 3 227 552, wobei das Verschleierungsmittel

auch diffusionsfest eingelagert sein kann.

Unverschleierte direktpositive Emulsionen sind beispielsweise solche, die im Innern der Silberhalogenidkörner Fehlstellen aufweisen (US 2 592 250) oder Silberhalogenidemulsionen mit geschichtetem Kornaufbau (DT—OS 2 308 239).

Wenn erfindungsgemäße farbgebende Verbindungen mit einem nichtdiffundierenden oxidierbaren Trägerrest eines solchen Typs verwendet werden, wie er in den DT—OSen 2 402 900 und 2 543 902 beschrieben ist, d.h. einen Trägerrest, der nur in nicht oxidierter Form durch Alkali abgespalten wird, während in oxidierter Form die Abspaltung erschwert oder verhindert ist, bedarf es selbstverständlich zur Herstellung positiver Übertragsbilder keiner direktpositiver Emulsionen und keiner Umkehrverfahren, sondern es genügen übliche Negativemulsionen.

Die Emulsionen können auch chemisch sensibilisiert werden, z.B. durch Zusatz schwefelhaltiger Verbindungen bei der chemischen Reifung, beispielsweise Allylisothiocyanat Allylthioharnstoff, Natriumthiosulfat und ähnliche. Als chemische Sensibilisatoren können ferner auch Reduktionsmittel, z.B. die in den belgischen Patentschriften 493 464 und 568 687 beschriebenen Zinnverbindungen, ferner Polyamine, wie Diäthylentriamin, oder Aminomethansulfinsäurederivate, z.B. geräß der belgischen Patentschrift 547 323 verwendet werden.

Geeignet als chemische Sensibilisatoren sind auch Edelmetalle bzw. Edelmetallverbindungen, wie Gold, Platin, Palladium, Iridium, Ruthenium oder Rhodium. Diese Methode der chemischen Sensibilisierung ist in dem Artikel von R. Koslowsky, Z. Wiss. Phot. 46, 65—72 (1951) beschrieben.

Es ist ferner möglich, die Emulsionen mit Polyalkylenoxid-derivaten zu sensibilisieren, z.B. mit Polyäthylenoxid eines Molekulargewichts zwischen 1000 und 20 000, ferner mit Kondensationsprodukten von Alkylenoxiden und aliphatischen Alkoholen, Glykolen, cyclischen Dehydratisierungsprodukten von Hexitolen, mit alkyl-substituierten Phenolen, aliphatischen Carbonsäuren, aliphatischen Aminen, aliphatischen Diaminen und Amiden. Die Kondensationsprodukte haben ein Molekulargewicht von mindestens 700, vorzugsweise von mehr als 1000. Zur Erzielung besonderer Effekte kann man diese Sensibilisatoren selbstverständlich kombiniert verwenden, wie in der belgischen Patentschrift 537 278 und in der britischen Patentschrift 727 982 beschrieben.

Die Emulsionen können auch spektral sensibilisiert sein, z.B. durch die üblichen Mono- oder Polymethinfarbstoffe, wie saure oder basische Cyanine, Hemicyanine, Streptocyanine, Merocyanine, Oxonole, Hemioxonole, Styrylfarbstoffe oder andere, auch drei- oder mehrkernige Methinfarbstoffe, beispielsweise Rhodacyanine oder Neocyanine. Derartige Sensibilisatoren sind beispielsweise beschrieben in dem Werk von F. M. Hamer "The Cyanine Dyes and Related Compounds" (1964) Interscience Publishers John Wiley and Sons.

Die Emulsionen können die üblichen Stabilisatoren enthalten, wie z.B. homöopolare oder salzartige Verbindungen des Quecksilbers mit aromatischen oder heterocyclischen Ringen, wie Mercaptotriazole, einfache Quecksilbersalze, Sulfoniumquecksilberdoppelsalze und andere Quecksilberverbindungen. Als Stabilisatoren sind ferner geeignet Azaindene, vorzugsweise Tetra- oder Pentaazaindene, insbesondere solche, die mit Hydroxyl- oder Aminogruppen substituiert sind. Derartige Verbindungen sind in dem Artikel von Birr. Z. Wiss. Phot. 47, 2—27 (1952) beschrieben. Weitere geeignete Stabilisatoren sind u.a. heterocyclische Mercaptoverbindungen, z.B. Phenylmercaptotetrazol, quaternäre Benzthiazolderivate, Benztriazol und ähnliche.

Als Bindemittel für die fotografischen Schichten wird vorzugsweise Gelatine verwendet, Diese kann jedoch ganz oder teilweise durch andere natürliche oder synthetische Bindemittel ersetzt werden. An natürlichen Bindemitteln sind z.B. Alginsäure und deren Derivate, wie Salze, Ester oder Amide, Cellulosederivate, wie Carboxymethylcellulose, Alkylcellulose, wie Hydroxyäthylcellulose, Stärke oder deren Derivate, wie Äther oder Ester oder Caragenate geeignet. An synthetischen Bindemitteln seien erwähnt Polyvinylalkohol, teilweise verseiftes Polyvinylacetat, Polyvinylpyrrolidon und dergleichen Die Härtung der Schichten kann in der üblichen Weise erfolgen, beispielsweise mit Formaldehyd oder halogensubstituierten Aldehyden, die eine Carboxylgruppe enthalten, wie Mucobromsäure, Diketonen, Methansulfonsäureester, Dialdehyden.

Zur Durchführung des Farbstoffdiffusionsübertragungsverfahrens, gemäß der vorliegenden Erfindung, wird ein lichtempfindliches Element verwendet, das eine oder mehrere Silberhalogenidemulsionsschichten, sowie die diesen zugeordneten nichtdiffundierenden, farbgebenden Verbindungen enthält, und ein Bild — empfangselement, in dem durch die bildmäßig übertragenen, diffundierenden Farbstoffe das gewünschte Farbbild erzeugt wird. Hierzu ist es erforderlich, daß zwischen dem lichtempfindlichen Element und dem Bildempfangselement midestens während eines endlichen Zeitraumes innerhalb der Entwicklungszeit ein fester Kontakt besteht, so daß die in dem lichtempfindlichen Element als Folge der Entwicklung erzeugte bildmäßige Verteilung an diffundierenden Farbstoffen auf das Bildempfangselement übertragen werden kann. Der Kontakt kann hergestellt werden, nachdem die Entwicklung in Gang gesetzt worden ist, oder er kann bereits hergestellt worden sein, bevor die Entwicklung beginnt. Letzteres ist beispielsweise der Fall, falls zur Durchführung des Farbstoffdiffusionsübertragungsverfahren ein Material verwendet wird, in dem das lichtempfindliche Element und das Bildempfangselement eine integrale Einheit bilden, im folgenden auch als Monoblattmaterial bezeichnet, die auch nach Beendigung des Entwicklungsvorganges weiter bestehen bleibt; d.h., eine Abtrennung des lichtempfindlichen Elementes vom Bildempfangselement ist auch nach

erfolgtem Farbübertrag nicht vorgesehen. Eine solche Ausführungsform ist beispielsweise beschrieben in der DT—OS 2 019 430.

Ein für die Durchführung des Farbstoffdiffusionsübertragungsverfahren gemäß der vorliegenden Erfindung geeignetes Monoblattmaterial weist beispielsweise folgende Schichtelemente auf:

1) einen transparenten Schichtträger
2) eine Bildempfangsschicht
3) eine lichtundurchlässige Schicht
4) ein lichtempfindliches Element mit mindestens einer lichtempfindlichen Silberhalogenidemulsionsschicht und mindestens einer dieser zugeordneten nichtdiffundierenden, farbgebenden Verbindung
5) eine Verzögerungsschicht
6) eine saure Polymerschicht
7) einen transparenten Schichtträger

Das Monoblattmaterial kann dabei in der Weise zusammengesetzt werden, daß getrennt voneinander zwei verschiedene Teile hergestellt werden, nämlich der lichtempfindliche Teil (Schichtelemente 1—4) und das Abdeckblatt (Schichtelemente 5—7), die dann schichtseitig aufeinander gelegt und miteinander verbunden werden, gegebenenfalls unter Verwendung von Abstandsstreifen, so daß zwischen den beiden Teilen ein Raum für die Aufnahme einer genau bemessenen Menge einer Arbeitsflüssigkeit gebildet wird. Die Schichtelemente 5 und 6, die zusammen das Neutralisationssystem bilden, können auch, allerdings in vertauschter Reihenfolge, zwischen dem Schichtträger und der Bildempfangsschicht des lichtempfindlichen Teiles angeordnet sein.

Es können Mittel vorgesehen sein, um eine Arbeitsflüssigkeit zwischen den lichtempfindlichen Teil und das Abdeckblatt einzuführen, z.B. in Form eines seitlich angeordneten, aufspaltbaren Behälters, der bei Einwirkung mechanischer Kräfte seinen Inhalt zwischen zwei benachbarte Schichten des Monoblattmaterials ergießt.

Ein wesentlicher Teil des fotografischen Materials gemäß der vorliegenden Erfindung ist das lichtempfindliche Element, das im Falle eines Einfarbstoffübertragungsverfahrens eine lichtempfindliche Silberhalogenidemulsionsschicht und dieser zugeordnet, eine nichtdiffundierende farbgebende Verbindung enthält. Dabei kann sich die nichtdiffundierende Verbindung in einer zu der Silberhalogenidemulsionsschicht benachbarten Schicht, oder in der Silberhalogenidemulsionsschicht selbst befinden, wobei im letzteren Fall die Farbe des Bildfarbstoffes bevorzugt so ausgewählt wird, daß der überwiegende Absorptionsbereich der farbgebenden Verbindung nicht übereinstimmt mit dem übelwiegenden Empfindlichkeitsbereich der Silberhalogenidemulsionsschicht. Zur Herstellung mehrfarbiger Übertragsbilder in naturetreuen Farben enthält das lichtempfindliche Element jedoch drei derartige Zuordnungen von farbgebender Verbindung und lichtempfindlicher Silberhalogenidemulsionsschicht, wobei in der Regel der Absorptionsbereich der farbgebenden Verbindung mit dem Bereich der spektralen Empfindlichkeit der zugeordneten Silberhalogenidemulsionsschicht im wesentlichen übereinstimmen wird. Vorraussetzung für eine möglichst hohe Empfindlichkeit ist dann jedoch, daß jeweils die farbgebende Kombination in einer separaten Bindemittelschicht (gesehen in Richtung des bei der Belichtung einfallenden Lichtes) hinter der Silberhalogenidemulsionsschicht angeordnet ist.

Die bei der Entwicklung einer Silberhalogenidemulsion entstehenden Entwickleroxidationsprodukte dürfen sich selbstverständlich nur auf die zugeordnete farbgebende Verbindung auswirken. Es sind deshalb im allgemeinen in dem lichtempfindlichen Element Trennschichten vorhanden, die die Diffusion der Entwickleroxidationsprodukte in andere nicht zugeordnete Schichten wirksam unterbinden. Diese Trennschichten können z.B. geeignete Substanzen enthalten, die mit den Entwickleroxydationsprodukten reagieren, beispielsweise nichtdiffundierende Hydrochinonderivate, oder falls es sich bei dem Entwickler um eine Farbentwicklersubstanz handelt, nichtdiffundierende Farbkuppler. In einer bevorzugten Ausführungsform hat deshalb das lichtempfindliche Element folgenden Aufbau (von oben nach unten):

Blauempfindliche Silberhalogenidemulsionsschicht
Schicht mit nichtdiffundierender, einen diffundierenden Gelbfarbstoff freisetzender Verbindung.
Trennschicht,
Grünsensibilisierte Silberhalogenidemulsionsschicht,
Schicht mit nichtdiffundierender, einen diffundierenden Purpurfarbstoff freisetzender Verbindung
Trennschicht
Rotsensibilisierte Silberhalogenidemulsionsschicht,
Schicht mit nichtduffundierender, einen diffundierenden Blaugrünfarbstoff freisetzender Verbindung.

Selbstverständlich können die Silberhalogenidemulsionsschichten auch in anderer Reihenfolge angeordnet sein, wobei jedoch die zugeordneten Schichten mit den farbgebenden Systemen ebenfalls vertauscht werden müssen, so daß die Zuordnung erhalten bleibt.

Die unter dem lichtempfindlichen Element angeordnete lichtundurchlässige Schicht ist durchlässig für wäßrige alkalische Behandlungslösungen und damit für die diffundierenden Farbstoffe. Sie hat im wesentlichen zwei Funktionen.

Erstens dient sie dazu, das nach der Entwicklung in dem ursprünglichen lichtempfindlichen

Element verbleibende Bildsilber, sowie die als Farbnegativ zurückbleibenden farbgebenden Verbindungen abzudecken, so daß bei der Betrachtung durch den transparenten Schichtträger des lichtempfindlichen Teils nur das positive Farbübertragsbild sichtbar ist. Zweitens schließt sie das lichtempfindliche Element nach der Seite der Bildempfangsschicht (nach unten) lichtdicht ab. Letzteres ist besonders dann von Bedeutung, wenn das Monoblattmaterial nach der Belichtung noch in der Kamera mit der alkalischen Verarbeitungsmasse in Berührung gebracht, dann aus der Kamera herausgezogen und außerhalb der Kamera entwickelt werden soll.

Schichten mit genügender Lichtundurchlässigkeit, aber genügender Durchlässigkeit für diffundierende Farbstoffe können beispielsweise mit Suspensionen anorganischer oder organischer dunkler, vorzugsweise schwarzer Pigmente, beispielsweise mit Suspensionen von Ruß in geeigneten Bindemitteln z.B. in Gelatinelösungen hergestellt werden. Im allgemeinen genügen 0,5 bis 2 $\mu$ starke Schichten, die in Gelatine 10 bis 90 Gew.-% (bezogen auf das gesamte Trockengewicht) an Ruß enthalten, um in genügendem Maße während der Entwicklung einen Ausschluß des Lichtes zu gewährleisten. Die Teilchengröße der verwendeten Pigmente ist relativ unkritisch, solange sie 0,5 $\mu$ nicht wesentlich überschreitet.

Die lichtundurchlässige Schicht umfaßt vorzugsweise außer der schwarzen Pigmentschicht noch eine darunter angeordnete weiße Pigmentschicht. Diese hat die Aufgabe, die schwarze Schicht zu verdecken und für das Bild einen weißen Untergrund zu schaffen. Geeignet hierfür sind alle weißen Pigmente, sofern ihre Deckkraft bei nicht allzu großen Schichtdicken genügend hoch ist. Erwähnt seien beispielsweise Bariumsulfat, Oxide von Zink, Titan, Silizium, Aluminium und Zirkon, ferner Bariumstearat oder Kaolin. Titandioxyd wird als weißes Pigment bevorzugt. Auch hierfür gelten hinsichtlich des Bindemittels, der Konzentration sowie der Teilchengröße die gleichen Angaben wie für die schwarzen Pigmente. Die Dicke der weißen Pigmentschicht kann je nach der gewünschten Weiße des Untergrundes variiert werden. Bevorzugt werden Dicken zwischen 5—20 $\mu$ eingesetzt.

Anstelle der lichtundurchlässigen Schicht können in dem Monoblattmaterial, gemäß der vorliegenden Erfindung, auch Mittel zur Erzeugung einer solchen lichtundurchlässigen Schicht zwischen dem lichtempfindlichen Element und der Bildempfangsschicht angeordnet sein, z.B. in Form eines seitlich angeordneten Behälters mit einer ein Trübungsmittel (Pigment) enthaltenden Arbeitsflüssigkeit, der bei Einwirkung mechanischer Kräfte seinen Inhalt zwischen die genannten Schichten ergießt, so daß sich dort eine derartige Pigmentschicht bildet.

Die Bildempfangsschicht besteht im wesentlichen aus einem Bindemittel, das Farbstoffbeizmittel für die Festlegung der diffundierenden Farbstoffe enthält.

Als Beizmittel für saure Farbstoffe dienen vorzugsweise langkettige quaternäre Ammonium- oder Phosphoniumverbindungen oder ternäre Sulfoniumverbindungen, z.B. solche wie sie beschrieben sind in den amerikanischen Patentschriften 3 271 147 und 3 271 148. Ferner können auch bestimmte Metallsalze und deren Hydroxyde, die mit den sauren Farbstoffen schwerlösliche Verbindungen bilden, verwandt werden. Die Farbstoffbeizmittel sind in der Empfangsschicht in einem der üblichen hydrophilen Bindemittel dispergiert, z.B. in Gelatine, Polyvinylpyrrolidon, ganz oder partiell hydrolysierten Celluloseesten und dergleichen. Selbstverständlich können auch manche Bindemittel als Beizmittel fungieren, z.B. Mischpolymerisate oder Polymerisatgemische von Vinylalkohol und N-Vinylpyrrolidon wie beispielsweise beschrieben in der DT—AS 1 130 284, ferner solche, die Polymerisate von stickstoffhaltigen quarternären Basen darstellen, z.B. Polymerisate von N-Methyl-2-vinylpyridin, wie beispielsweise beschrieben in der amerikanischen Patentschrift 2 484 430. Weitere brauchbare beizende Bindemittel sind beispielsweise Gyanylhydrazonderivate von Acylstyrol-polymerisaten wie beispielsweise beschrieben in der DT—OS 2 009 498. Im allgemeinen wird man jedoch den zuletzt genannten beizenden Bindemitteln andere Bindemittel, z.B. Gelatine zusetzen.

Als transparente Schichtträger für das erfindungsgemäße Monoblattmaterial können die üblichen in der photographischen Praxis verwendeten transparenten Trägermaterialien, z.B. Filme aus Celluloseestern, Polyäthylenterephthalat, Polycarbonat oder anderen filmbildenden Polymeren, Verwendung finden.

Durch die alkalische Verarbeitungsmasse wird in dem lichtempfindlichen Material ein realtiv hoher pH-Wert eingestellt (etwa 11 bis 14), wodurch die Entwicklung und die bildmäßige Farbstoffdiffusion ausgelöst wird. Es hat sich erwiesen, daß bei diesem hohen pH-Wert die Farbstoffe und damit die erhaltenen Bilder nicht sonderlich stabil sind. Es ist daher erforderlich, daß nach vollendeter Entwicklung das Material nahezu neutral oder schwachsauer gestellt wird. Das kann in bekannter Weise dadurch erreicht werden, daß das Material zusätzlich eine saure Polymerschicht enthält, die erst allmählich im laufe der Entwicklung für die alkalische Verarbeitungsmasse zugänglich wird. Unter einer sauren Polymerschicht wird eine Bindemittelschicht verstanden, die polymere Verbindungen mit Säuregruppen, vorzugsweise Sulfo- oder Carboxylgruppen enthält. Diese sauren Gruppen reagieren mit den Kationen der Verarbeitungsmasse unter Salzbildung und erniedrigen hierbei den pH-Wert der Masse. Selbstverständlich sind die polymeren Verbindungen und damit die sauren Gruppen in der genannten Schicht diffusionsfest eingelagert. Vielfach stellen die sauren Polymeren Derivate der Cellulose oder Derivate von Polyvinylverbindungen dar, es können jedoch auch andere polymere Verbindungen Verwendung finden. Als brauchbare saure Polymere seien beispielsweise erwähnt: Cellulosederivate mit freier Carboxylgruppe, z.B. Cellulosedicarbonsäurehalbester mit freier Carboxylgruppe wie Cellulose-

acetathydrogenphthalat, Celluloseacetathydrogenglutarat, Äthylcelluloseacetathydrogensuccinat, Celluloseacetathydrogensuccinathydrogenphthalat, Äther und Ester von Cellulose, die mit weiteren Dicarbonsäureanhydriden oder mit Sulfonsäureanhydriden, beispielsweise mit o-Sulfobenzoesäureanhydrid modifiziert sind, Carboxymethylcellulose, ferner Polystyrolsulfonsäure, Polyvinylhydrogenphthalat, Polyvinylacetathydrogenphthalat, Polyacrylsäure, Acetale von Polyvinylalkohol mit Aldehyden, die durch Carboxy- oder Sulfogruppen substituiert sind, wie o-, m- oder p-Benzaldehydsulfonsäure oder -carbonsäure, partiell veresterte Äthylen-Maleinsäureanhydrid-Mischpolymerisate, partiell veresterte Methylvinyläther-Maleinsäureanhydrid-Mischpolymerisate und dergleichen.

Die saure Polymerschicht muß genügend Säuregruppen enthalten, um den pH-Wert der Verarbeitungsmasse von anfangs 11 bis 14 so weit zu erniedrigen, daß das Material zum Schluß nahezu neutral oder schwach sauer eingestellt ist (pH-Wert 5 bis 8).

Die zeitliche Verzögerung der pH-Werterniedrigung wird in bekannter Weise dadurch erreicht, daß die saure Polymerschicht mit einer sogenannten Verzögerungsschicht überzogen ist. Diese Verzögerungsschicht stellt eine alkalidurchlässige Schicht dar, die vorzugsweise aus einem gegen Alkali inerten Polymeren, beispielsweise aus Polyvinylalkohol oder einem partiell acetalisierten Polyvinylalkohol besteht. Durch geeignete Wahl der Dicke und der Zusammensetzung dieser Verzögerungsschicht kann die zeitliche Verzögerung der pH-Werterniedrigung in der gewünschten Weise eingestellt werden. Eine bremsschicht mit Polymeren eines neuartigen Durchlässigkeitsverhalten ist beispielsweise in der DT—OS 2 455 762 beschrieben.

Neutralisationssysteme, worunter Kombination aus einer sauren Polymerschicht und einer Verzögerungsschicht verstanden werden, sind beispielsweise beschrieben in der DT—PS 1 285 310. Derartige Schichtkombinationen können in dem erfindungsgemäßen Material vorhanden sein, beispielsweise in dem lichtempfindlichen Teil zwischen dem transparenten Schichtträger und der Bildempfangsschicht. Eine weitere Möglichkeit besteht darin, das Neutralisationssystem aus einer sauren Polymerschicht und einer Verzögerungsschicht auf dem Deckblatt anzuordnen. Selbstverständlich müssen diese beiden Schichten in einer derartigen Reihenfolge angeordnet sein, daß das Alkali der Verarbeitungsmasse zunächst die Verzögerungsschicht durchdringen muß, um in die saure Polymerschicht zu gelangen.

Das erfindungsgemäße Farbstoffdiffusionsübertragungsverfahren läßt sich vorteilhaft in bzw. mittels einer geeigneten Selbstentwicklerkamera durchführen. Diese kann z.B. mit Vorrichtungen versehen sein, die es ermöglichen, nach der Belichtung des lichtempfindlichen Elementes zwischen diesem und dem Deckblatt eine Arbeitslösung zu verteilen und das lichtempfindliche Material nach oben lichtundurchlässig abzudecken. Vorzugsweise ist eine solche Kamera mit zwei gegeneinander gelagerten Quetschwalzen versehen, zwischen denen das Monoblattmaterial herausgezogen wird, wobei die seitlich angeordneten Behälter aufgespalten werden und ihren Inhalt zwischen die Schichten des Monoblattmaterials entlassen.

Da das lichtempfindliche Element nach dem Passieren der Quetschwalzen beiderseits durch lichtundurchlässige Schichten geger unerwünschte Belichtung geschützt ist, kann das belichtete Material unmittelbar nach Ingangsetzung der Entwicklung aus der Kamera herausgezogen werden.

Zur Verarbeitung des bildmäßig belichteten Monoblattmaterials wird das lichtempfindliche Element in Kontakt gebracht mit der wäßrig alkalischen Arbeitslösung. Hierbei werden in Gegenwart der Entwicklerverbindung die bildmäßig belichteten Silberhalogenidemulsionsschichten entwickelt, wobei in Übereinstimmung mit dem dabei gebildeten positiven Silberbild eine bildmäßige Verteilung von Oxydationsprodukten der Entwicklerverbindung erzeugt wird, die die zugeordnete farbgebende Verbindung oxydiert, worauf diese unter Reaktion mit dem Alkali des Aktivators den diffundierenden Farbstoff abspaltet. Die wäßrig alkalische Arbeitslösung kann viskositätserhöhende Zusätze enthalten, z.B. Hydroxyäthylcellulose. Weiterhin können in der Arbeitslösung in bekannter Weise Entwicklungsbeschleuniger, Stabilisatoren, Silbersaltzlösungsmittel, Schleiermittel, Antioxydantien und weitere Zusätze enthalten sein.

## Beispiel 1

Ein lichtempfindliches Element eines fotografischen Materials gemäß der Erfindung wurde dadurch hergestellt, daß auf einem transparenten Träger aus Polyesterfolie folgende Schichten nacheinander aufgetragen wurden. Die Mengenangaben beziehen sich dabei jeweils auf 1 $m^2$.

1) Eine Beizschicht aus 6 g eines Polyurethans aus 4,4-Diphenylmethandiisocyanat, N-Äthyldiäthanolamin und Epichlorhydrin und 6,0 g Gelatine.
2) Eine Reflektionsschicht aus 24 g $TiO_2$ und 2,4 g Gelatine.
3) Eine Rußschicht aus 1,9 g Ruß und 2 g Gelatine.
4) Eine Farbstoffschicht aus 0,5 g der Verbindung I (blaugrün) und 0,9 g Gelatine.
5) Eine rot sensibilisierte Emulsionsschicht mit einer unverschleierten direkt-positiv arbeitenden Silberchloridbromidemulsion, Silberauftrag 2,6 g, Gelatine 1,3 g und 0,04 g N-[$\alpha$-(2,4-ditert,-amylphenoxy)-acetyl]-N'-phenyl-hydrazin als Schleiermittel.
6) Eine Sperrschicht aus 0,5 g Octadecylhydrochinonsulfosäure und 1,3 g Gelatine.
7) Eine Farbstoffschicht aus 0,5 g der Verbindung II (purpur) und 0,9 g Gelatine.
8) Eine grün sensibilisierte Emulsionsschicht mit einer unverschleierten direkt positiv arbeitenden

Silberchloridbromidemulsion, Silberauftrag 2,5 g, Gelatine 1,28 g und 0,04 g N-[$\alpha$-(2,4-ditert.-amylphenoxy)-acetyl]-N'-phenyl-hydrazin als Schleiermittel.

9) Eine Sperrschicht identisch mit Schicht 6).

10) Eine Farbstoffschicht aus 0,65 g der Verbindung III (gelb) und 1,0 g Gelatine.

11) Eine blau sensibilisierte Emulsionsschicht mit einer unverschleierten direkt-positiv arbeitenden Silberchloridbromidemulsion, Silberauftrag 2,7 g, Gelatine 1,4 g und 0,04 g N-[$\alpha$-(2,4-ditert.-amylphenoxy)-acetyl]-N'-phenyl-hydrazin als Schleiermittel.

12) Eine Schutzschicht aus 0,8 g Gelatine und 0,8 g einer Verbindung der folgenden Formel (Härtungsmittel):

$$C_2H_5—N=C=N—CH_2—CH_2—CH_2—\overset{\oplus}{N}(C_2H_5)_3 \quad \overset{\ominus}{Cl}$$

13) Ein transparentes Deckblatt aus Polyäthylenterephthalat mit einer Neutralisationsschicht und einer Verzögerungsschicht.

Nach Belichtung durch einen Stufenkeil wurde das lichtempfindliche Element mit dem transparenten Deckblatt auf der Schichtseite abgedeckt. Zur Entwicklung des bildmäßig belichteten lichtempfindlichen Elements wurde eine aufspaltbarer Behälter mit einer alkalischen Arbeitsflüssigkeit folgender Zusammensetzung verwendet

50 g KOH
10 ml Benzylalkohol
3 g Benzotriazol
10 g Natriumsulfit
6,0 g 1-Phenyl-4-methyl-4-hydroxymethyl-3-pyrazolidon
0,1 g Hydrochinon
34 g Hydroxyäthylcellulose
aufgefüllt auf 1000 ml Wasser.

Der Bildset wurde durch ein Quetschwalzenpaar geführt, wobei sich die Entwicklerpaste zwischen lichtempfindlichem Element und Abdeckblatt verteilte. Die Pastenstärke betrug 110 $\mu$. Um diese einzustellen, wurden seitlich entland dem Bildrand zwischen lichtempfindlichem Element und Abdeckblatt Abstandsstreifen entsprechender Dicke angebracht.

Weitere lichtempfindliche Elemente wurden in der gleichen Weise hergestellt mit der einzigen Aus nahme, daß der Farbstoff III der Schicht durch eine der erfindungsgemäßen Verbindungen Nr. 3 bis Nr. 7 ersetzt wurde. Nach gleicher Verarbeitung resultierten ebenfalls direkt-positive mehrfarbige Abbildungen der Vorlage (Filmsets B—F).

Nach einer Entwicklungszeit von 1 Stunde wurden mittels eines Reflektionsdensitometers unter Verwendung eines Blaufilters die in der folgenden Tabelle aufgeführten Dmax-und Dmin-Werte der Bildempfangsschichten ermittelt.

TABELLE

| Filmset | Verb. | Dmax | Dmin |
|---|---|---|---|
| A | III | 1,65 | 0,32 |
| B | 3 | 1,70 | 0,29 |
| C | 4 | 1,52 | 0,32 |
| D | 5 | 1,92 | 0,31 |
| E | 6 | 1,71 | 0,29 |
| F | 7 | 1,98 | 0,30 |

Aus den erhaltenen Ergebnissen ist ersichtlich, daß mit den farbgebenden Verbindungen der vorliegenden Erfindung höhere Übertragsfarbdichten zu erreichen sind.

Formelanhang zu Beispiel 1

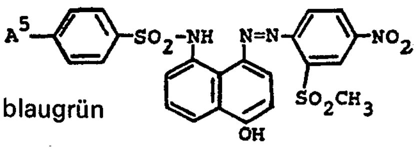

blaugrün I

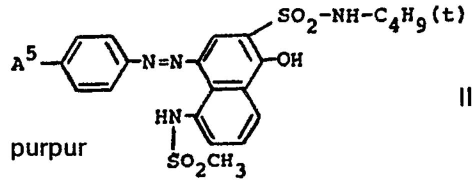

purpur II

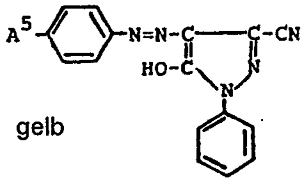

gelb III

Die Prüfung der Lichtechtheit und die Messung der Absorption wurde an den Übertragsfarbstoffen selbst vorgenommen, die aus den farbgebenden Verbindungen in diffundierender Form freigesetzt werden.

Der zu untersuchende Farbstoff wurde durch kontinuierliches Eintauchen eines transparenten Schichtträgers mit einer Beizschicht, die der Schicht 1 aus Beispiel 1 entspricht, in eine wäßrige Farbstofflösung aufgebeizt. Anschließend wurde 2 Minuten gewässert und getrocknet.

Die Absorptionsmessung wurde mit einem Spektralphotometer durchgeführt.

Zur Prüfung der Lichtechtheit wurde je ein Streifen der erhaltenen Materialien mit den zu vergleichenden Farbstoffen auf einer $TiO_2$-Unterlage befestigt und 24 Stunden lang im Xenotestgerät der Fa. Hanau einer Bestrahlung von $1.10^5$ 1x ausgesetzt (ca. 20°C; ca. 60% rel. Luftfeuchtigkeit). Die nach der Bestrahlung verbleibende Restdichte wurde nach folgender Gleichung berechnet.

$$\text{Restdichte} = \frac{\text{Farbdichte nach Belichtung}}{\text{Farbdichte vor Belichtung}} \cdot 100\ [\%]$$

Die Messung erfolgte im Dichtebereich (vor Belichtung) von 1,0 bis 1,5.
Die erhaltenen Restdichten sind der folgenden Tabelle zu entnehmen.

Tabelle

| Farbstoff aus Verb. | Restdichte [%] |
|---|---|
| 1 | 63 |
| 2 | 69 |
| 3 | 74 |
| 4 | 78 |
| 5 | 70 |
| 6 | 67 |
| 7 | 77 |
| 8 | 71 |
| 9 | 75 |
| 10 | 75 |
| III (Vergleich) | 34 |

**Patentansprüche**

1. Fotografisches Farbdiffusionsübertragungsverfahren zur Herstellung farbiger Bilder, bei dem ein fotografisches Material mit mindestens einer lichtempfindlichen Silberhalogenidemulsionsschicht und einer dieser zugeordneten nichtdiffundierenden farbgebenden Verbindung bildmäßig belichtet und mit einem Silberhalogenidentwicklungsmittel entwickelt wird, wobei durch das Entwickleralkali bildmäßig ein diffundierrender Farbstoff aus der nichtdiffundierenden farbgebenden Verbindung abgespalten und auf eine Bildempfangsschicht übertragen wird, dadurch gekennzeichnet, daß als nichtdiffundierende farbgebende Verbindung eine Verbindung der folgenden Formel I verwendet wird:

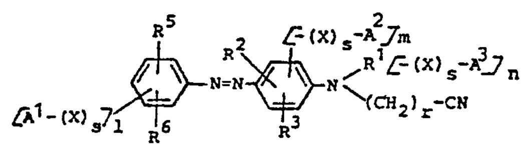

(I)

worin bedeuten

$A^1$ einen gegebenenfalls über ein Bindeglied X angeknüpften einen diffusionsfestmachenden Rest enthaltenden oxidierbaren organischen Trägerrest in o-, m- oder p-Stellung zur Azogruppe, aus dem unter den Bedingungen der fotografischen Entwicklung entweder in oxidierter Form oder in nicht oxidierter Form durch Entwickleralkali mindestens ein Teil zusammen mit dem diffusionsfestmachenden Rest abgespalten wird unter gleichzeitiger bildmäßiger Freisetzung eines diffundierenden Azofarbstoffes;

$A^2$ einen gegebenenfalls über ein Bindeglied X angeknüpften einen diffusionsfestmachenden Rest enthaltenden oxidierbaren organischen Trägerrest, der entweder direkt oder über einen der Substituenten $R^2$ und $R^3$ in o- oder m-Stellung an den die Cyanalkylaminogruppe tragenden Phenylring angeknüpft ist und aus dem unter den Bedingungen der fotografischen Entwicklung entweder in oxidierter Form oder nicht oxidierter Form durch Entwickleralkali mindestens ein Teil zusammen mit dem diffusionsfestmachenden Rest abgespalten wird unter gleichzeitiger bildmäßiger Freisetzung eines diffundierenden Azofarbstoffes,

$A^3$ einen gegebenenfalls über ein Bindeglied X angeknüpften, einen diffusionsfestmachenden Rest enthaltenden oxidierbaren organischen Trägerrest, der mit dem Substituenten $R^1$ verknüpft ist und aus dem unter den Bedingungen der fotografischen Entwicklung entweder in oxidierter Form oder in nicht oxidierter Form durch Entwickleralkali mindestens ein Teil zusammen mit dem diffusionsfestmachenden Rest abgespalten wird, unter gleichzeitiger bildmäßiger Freisetzung eines diffundierenden Azofarbstoffs;

$R^1$ Wasserstoff, Alkyl mit 1 bis 5 C-Atomen, Aralkyl oder Aryl

$R^2$, $R^3$, $R^5$ und $R^6$ (gleich oder verschieden), je einen der unter $R^1$ genannten Reste, Sulfo, —$COOR^4$, Halogen, Trihalogenmethyl, Acylamino, Acyloxy, Carbamoyl, Sulfamoyl, —$OR^4$, —$SR^4$, —$SOR^4$, —$SO_2$—$R^4$ (wobei $R^4$ eine der für $R^1$ angegebenen Bedeutung hat), —$NO_2$ oder —CN

25 X ein bivalentes Bindeglied der Formel $—R—(L)_p—(R)_q—$, worin R einen Alkylenrest mit 1 bis 6 C-Atomen oder einen gegenbenfalls substituierten Phenylenrest bedeutet und wobei die beiden Reste R gleiche oder verschiedene Bedeutungen haben können;

L —O—, —CO—, —$CONR^7$, —$SO_2NR^7$, —O—CO—$NR^7$, —S—, —$SO_2$—, —SO— bedeutet ($R^7$ = Wasserstoff oder Alkyl)

$p = 0$ oder 1

$q = 0$ oder 1, wobei $q = 1$, falls $p = 1$

l, m und n jeweils 0 oder 1 mit der Maßgabe, daß

$1 + m + n = 1$

r 2 oder 3

s 0 oder 1.

2. Verfahren nach Anspruch 1, dadurch gekennzeichnet, daß als nichtdiffundierende farbgebende Verbindung eine Verbindung der folgenden Formel II verwendet wird:

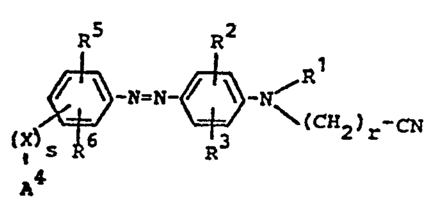

(II)

worin bedeuten

$R^1$ Wasserstoff oder Alkyl mit 1 bis 5 C-Atomen,

$R^2$, $R^3$, $R^5$ und $R^6$ (gleich oder verschieden) Wasserstoff, Chlor, Methyl, Methoxy, Acylamino, Nitro,

$A^4$ den mit mindestens einem diffusionsfestmachenden Rest substituierten Rest einer der folgenden Formeln

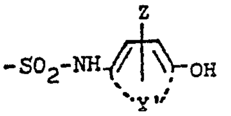

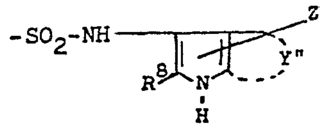

worin

Z für einen diffusionsfestmachenden Rest steht,

Y′ für einen zur Vervollständigung eines Benzol- oder Naphthalinringes erforderlichen Rest steht,

Y″ für einen zur Vervollständigung eines ankondensierten, gegebenenfalls substituierten carbocyclischen oder heterocyclischen Ringes steht, und

$R^8$ Wasserstoff, Alkyl, Alkoxy, Aryl, eine heterocyclische Gruppe, Carboxyl, Carbamoyl oder Alkoxycarbonyl bedeutet,

X ein bivalentes Bindeglied der Formel $—R—(L)_p—(R)_q—$,

worin

R einen Alkylenrest mit 1 bis 6 C-Atomen oder einen gegebenenfalls substituierten Phenylenrest bedeutet und wobei die beiden Reste R gleiche oder verschiedene Bedeutungen haben können;

L —O—, —CO—, —$CONR^7$, —$SO_2NR^7$, —O—CO—$NR^7$, —S—, —$SO_2$—, —SO— bedeutet ($R^7$ = Wasserstoff oder Alkyl),

p 0 oder 1

q 0 oder 1, wobei q = 1, falls p = 1

r 2 oder 3

s 0 oder 1.

## Claims

1. A photographic dye diffusion transfer process for the production of coloured images, in which a photographic material having at least one light-sensitive silver halide emulsion layer and a non-diffusible dye-providing compound associated with this layer is exposed imagewise and developed with a silver halide developer, a diffusible dye being released imagewise from the non-diffusible dye-providing compound by the alkali of the developer and transferred to an image receptor layer, characterised in that the non-diffusible dye-providing compound used is a compound corresponding to the following formula I:

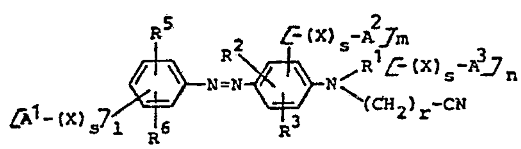

(I)

in which

$A^1$ represents an oxidizable organic carrier residue in the o-, m- or p-position to the azo group, which carrier residue contains a group which confers diffusion resistance and which carrier residue may be attached through a connecting member X, from which carrier residue, either in its oxidized form or in its unoxidized form, at least part thereof together with the group which confers diffusion resistance is split off by developer alkali under the conditions of photographic development and a diffusible azo dye is at the same time released imagewise;

$A^2$ represents an oxidizable organic carrier residue containing a group which confers diffusion resistance and which carrier residue may be attached through a connecting member X, which carrier residue is attached in the ortho- or meta-position, either directly or through one of the substituents $R^2$ and $R^3$, to the phenyl ring which carries the cyanoalkylamino group, from which carrier residue, either in the oxidized form or in the unoxidized form, at least part thereof together with the group which confers diffusion resistance is split off by developer alkali under the conditions of photographic development and at the same time a diffusible azo dye is released imagewise;

$A^3$ represents an oxidizable organic carrier residue which may be attached through a connecting member X and contains a group which confers diffusion resistance, which carrier residue is attached to the substituent $R^1$ and from which carrier residue, either in the oxidized form or in the unoxidized form, at least a part thereof together with the group which confers diffusion resistance is split off by developer alkali under the conditions of photographic development and a diffusible azo dye is at the same time released imagewise;

$R^1$ represents hydrogen, alkyl with 1 to 5 C atoms, aralkyl or aryl;

$R^2$, $R^3$, $R^5$ and $R^6$, which may be the same or different, each represents one of the groups mentioned under $R^1$, sulpho, —$COOR^4$, halogen, trihalogenmethyl, acylamino, acyloxy, carbamoyl, sulphamoyl, —$OR^4$, —$SR^4$, —$SOR^4$, —$SO_2$—$R^4$, wherein $R^4$ has one of the meanings specified for $R^1$, —$NO_2$ or —CN;

X represents a bivalent connecting member of the formula —R—$(L)_p$—$(R)_q$—, in which R represents an alkylene group with 1 to 6 C atoms or an optionally substituted phenylene group and the two groups R may be the same or different;

L represents —O—, —CO—, —$CONR^7$, —$SO_2NR^7$, —O—CO—$NR^7$, —S—, —$SO_2$— or —SO— in which $R^7$ = hydrogen or alkyl;

$p$ = 0 or 1,

$q$ = 0 or 1, and $q$ = 1 if $p$ = 1

$l$, $m$ and $n$ each represents 0 or 1 such that $l + m + n = 1$;

$r$ represents 2 or 3; and

$s$ represents 0 or 1.

2. The process as claimed in claim 1, characterised in that the non-diffusible dye-providing compound used is a compound of the following formula II:

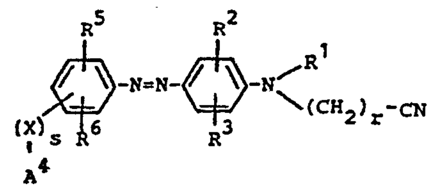

(II)

in which

$R^1$ represents hydrogen or alkyl with 1 to 5 C atoms

$R^2$, $R^3$, $R^5$ and $R^6$ which may be the same or different, represent hydrogen, chlorine, methyl, methoxy, acylamino, or nitro;

$A^4$ represents a group corresponding to one of the following formulae substituted with at least one group which confers diffusion resistance:

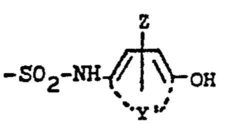

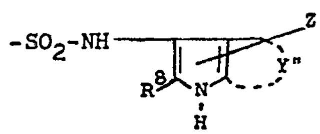

in which

Z represents a group which confers diffusion resistance;

Y' represents a group required to complete a benzene or naphthalene ring;

Y" represents a group required to complete a condensed, carbocyclic or heterocyclic ring, which may be substituted and

$R^8$ represents hydrogen, alkyl, alkoxy, aryl, a heterocyclic group, carboxyl, carbamoyl or alkoxycarbonyl;

X represents a bivalent connecting member corresponding to the formula —R—$(L)_p$—$(R)_q$—, in which

R represents an alkylene group with 1 to 6 C atoms or an optionally substituted phenylene group and the two groups R may be the same or different;

L represents —O—, —CO—, —$CONR^7$, —$SO_2NR^7$ —O—CO—$NR^7$ —S— —$SO_2$— or —SO— in which $R^7$ = hydrogen or alkyl;

$p$ = 0 or 1;

$q$ = 0 or 1; and $q$ = 1 if $p$ = 1

$r$ = 2 or 3 and

$s$ = 0 or 1.

**Revendications**

1. Procédé photographique couleur de diffusion-transfert pour la fabrication d'images en couleur, dans lequel un matériau photographique comportant au moins une couche d'émulsion d'halogénure d'argent sensible à la lumière et un composé non diffusible libérant un colorant et associé à cette couche est exposé suivant une image et développé au moyen d'un révélateur de l'halogénure d'argent, un colorant diffusible étant libéré suivant l'image par le composé chromogène sous l'action de l'alcali du révélateur et transféré dans une couche réceptrice d'image, ledit procédé étant caractérisé en ce que l'on utilise comme composé chromogène non diffusible un composé de formule générale:

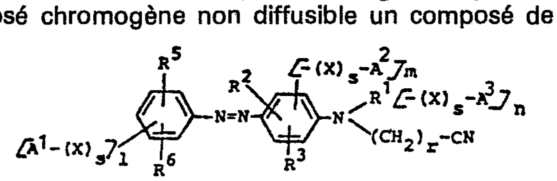

(I)

dans laquelle:

$A^1$ représente un reste organique de support oxydable en position o- m- ou p- par rapport au groupe azo, contenant un reste conférant la résistance à la diffusion et éventuellement lié par un élément de liaison X, dont au moins une portion est scindée dans les conditions du développement photographique, soit sous forme oxydée soit sous forme non oxydée, sous l'action de l'alcali du révélateur, conjointement avec le reste conférant la résistance à la diffusion, avec libération simultanée suivant l'image d'un colorant azoïque diffusible;

$A^2$ représente un reste organique de support oxydable contenant un reste qui confère la résistance à la diffusion et lié éventuellement par un élément de liaison X, ce reste de support étant relié soit directement soit par l'intermédiaire des substituants $R^2$ et $R^3$ en position o- ou m- au noyau phényle portant le groupe cyanoalkylamino, et au moins une portion est scindée dans les conditions du développement photographique, soit sous forme oxydée, soit sous forme non oxydée, sous l'action de l'alcali du révélateur, conjointement avec le reste conférant la résistance à la diffusion, avec libération simultanée suivant l'image d'un colorant azïque diffusible;

$A^3$ représente un reste organique de support oxydable contenant un reste conférant la résistance à la diffusion et lié, éventuellement par un groupe de liaison X, qui est lié au substituant $R^1$, dont au moins une portion est scindée dans les conditions du développement photographique, soit sous forme oxydée, soit sous forme non oxydée, sous l'action de l'alcali du révélateur, conjointement avec le reste conférant la résistance à la diffusion, avec libération simultanée suivant l'image d'un colorant azoïque diffusible;

$R^1$ reprèsente l'hydrogène ou un groupe alkyle en $C_1$—$C_5$, arylalkyle ou aryle,

$R^2$, $R^3$, $R^5$, et $R^6$ (identiques ou différents) représentent chacun un des restes mentionnés sous $R^1$, un halogène ou un groupe sulfo, —$COOR^4$, trihalogénométhyle, acylamino, acyloxy, carbamoyle, sulfamoyl, —$OR^4$, —$SR^4$, —$SOR^4$, —$SO_2R^4$ (où $R^4$ représente l'un des restes indiqués pour $R^1$) —$NO_2$ ou —CN,

X représente un élément de liaison bivalent de formule —R—$(L)_p$—$(R)_q$—,

dans laquelle R représente un reste alkylène en $C_1$—$C_6$ ou un reste phénylène éventuellement substitué et les deux restes R peuvent être identiques ou différents;

L représente —O—, —CO—, —$CONR^7$, —$SO_2NR^7$, —O—CO—$NR^7$, —S—, —$SO_2$—, —SO— ($R^7$ = hydrogène ou alkyle)

p = 0 ou 1

q = 0 ou 1, et q = 1 si p = 1

l, m et n sont chacun égal à 0 ou à 1 avec la condition que l + m + n = 1,

r est égal à 2 ou 3,

s est égal à 0 ou 1.

2. Procédé selon la revendication 1, caractérisé en ce que l'on utilise comme composé chromogène non diffusible un composé de formule suivante:

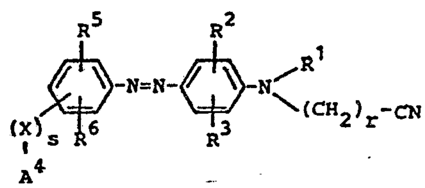

(II)

dans laquelle

$R^1$ représente l'hydrogène ou un groupe alkyle en $C_1$—$C_5$,

$R^2$, $R^3$, $R^5$ et $R^6$ (identiques ou différents) représentent l'hydrogène ou le chlore ou un groupe méthyle, méthoxy, acylamino, ou nitro,

$A^4$ représente le reste substitué par au moins un reste conférant la résistance à la diffusion, répondant à l'une des formules suivantes:

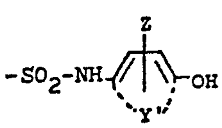

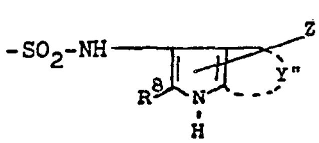

dans lesquelles

Z représente un reste conférant la résistance à la diffusion,

Y' représente un reste nécessaire pour compléter un noyau benzénique ou naphthalénique

Y" représente un reste nécessaire pour compléter un noyau carbocyclique ou hétérocyclique condensé, éventuellement substitué, et

$R^8$ représente l'hydrogène ou un groupe alkyle, alcoxy, aryle, un groupe hétérocyclique ou un groupe carboxy, carbamoyle ou alcoxycarbonyle,

X représente un élément de liaison bivalent de formule $—R—(L)_p—(R)_q—$, dans laquelle R représente un reste alkylène en $C_1—C_6$ ou un reste phénylène éventuellement substitué et les deux restes R peuvent être identiques ou différents;

L représente —O—, —CO—, $—CONR^7$, $—SO_2NR^7$, $—O—CO—NR^7$, —S—, $—SO_2—$, —SO— ($R^7$ = hydrogène ou alkyle)

p est égal à 0 ou à 1

q est égal à 0 ou à 1 et q = 1 si p = 1

r est égal à 2 ou à 3

s est égal à 0 ou à 1.